# EUROPEAN PATENT APPLICATION

(11) **EP 2 228 068 A1**
(43) Date of publication of application: **15.09.2010**
(21) Application number: 08865804.2
(22) Date of filing: 25.12.2008
(51) Int. Cl.: A61K 38/00, A61K 47/48, A61P 9/00, A61P 25/00, A61P 43/00

(54) **THERAPEUTIC AGENT FOR CEREBRAL ISCHEMIC INJURY**

(30) Priority: 26.12.2007 JP 2007335024
(71) Applicant: Otsuka Pharmaceutical Factory, Inc., Tokushima 772-8601 (JP)
(72) Inventor: KAWANO, Yuichi, Naruto-shi Tokushima 772-8601 (JP); DOI, Kazuhisa, Naruto-shi Tokushima 772-8601 (JP); NII, Takuya, Naruto-shi Tokushima 772-8601 (JP)
(74) Representative: Neuefeind, Regina
(86) International application number: PCT/JP2008/073654
(87) International publication number: WO 2009/081991

(57) **Abstract**

A therapeutic agent for cerebral ischemic injury contains at least one selected from L-alanyl-L-histidine and glycyl-L-histidine as an active ingredient. The therapeutic agent for cerebral ischemic injury preferably has a dosage form as an injection for intravenous administration. The therapeutic agent for cerebral ischemic injury having a dosage form as an aqueous injection contains at least one selected from L-alanyl-L-histidine and glycyl-L-histidine at a concentration of preferably 0.3 to 3.5 mol/L, and more preferably 0.6 to 2.1 mol/L.

## Description

### TECHNICAL FIELD

The present invention relates to a therapeutic agent for cerebral ischemic injury, and specifically to a therapeutic agent for cerebral ischemic injury that contains, as an active ingredient, an L-histidine derivative having a high solubility in a solvent.

### BACKGROUND ART

There have been known lifestyle-related diseases such as obesity, hypertension, hyperglycemia, and hyperlipidemia, which are developed by bad lifestyle habits such as ingestion of high-caloric diet, alcohol drinking, smoking, lack of exercise, and stress. Such lifestyle-related diseases are considered to cause adult diseases such as cerebral infarction and myocardial infarction. Among adult diseases, cerebral infarction is often attended with after effects such as physical disabilities, language disorder, and dementia, after the recovery of the disease under the present circumstances.

Cerebral infarction is a disease in which cerebral ischemia occurs, for example, due to occlusion of an artery of the brain by a thrombus or narrowing of an artery of the brain by hardening thereof, and the supply of oxygen and nutrition to the brain tissue thereby becomes insufficient, resulting in necrosis of the brain tissue or a state close to such necrosis. It has been known that, in such cerebral infarction, free radicals are generated when the brain has recovered from a cerebral ischemic condition, and such free radicals cause further necrosis to develop in peripheral brain tissue, and thus cerebral ischemic injury further progresses.

The treatment of cerebral ischemic injury is broadly classified into two types of treatment methods. In one treatment method, blood supply is reestablished, for example, by thrombolysis and vasodilatation. In the other treatment method, the progression of the necrosis of brain tissue due to free radicals or swelling is prevented. In both methods, pharmacotherapy is mainly applied. In the former method, a thrombolytic agent and a platelet aggregation inhibitor are used, for example. In the latter method, a free radical scavenger and an antihydropic agent are used, for example. In order to achieve a good prognosis for cerebral infarction, it is necessary to combine the two types of treatments, namely, the reestablishment of blood supply and the prevention of the progression of necrosis. Of these, the prevention of the progression of necrosis is particularly important.

However, agents for preventing the progression of necrosis to suppress brain disorder are limited, and these agents are problematic in terms of side effects. A free radical scavenger, Edaravone (3-methyl-1-phenyl-2-pyrazoline-5-one), has been known as a brain-protecting agent. However, such Edaravone is problematic in that it brings about side effects such as hepatic impairment and renal impairment at a high rate. Hence, a therapeutic agent for cerebral ischemic injury having a few side effects has been widely sought.

For example, Patent Document 1 describes that an amino acid, L-histidine, exhibits the effect of suppressing cerebral ischemic injury. However, L-histidine is problematic in that the solubility of free L-histidine in water is low (4.3 g/dL (0.28 mol/L) at 25°C). In order to rapidly increase the L-histidine concentration in blood, intravenous administration is desirable. However, since the solubility of L-histidine in water is low, it is difficult to prepare an injection containing L-histidine at an effective concentration. In order to increase the solubility of L-histidine, it is considered necessary to use L-histidine in the form of a salt such as hydrochloride or to add acid to an injection to make it acidic. In such cases, however, there is a risk of causing side effects such as acidosis.

Patent Document 1: International Publication No. WO2006/025598

### DISCLOSURE OF THE INVENTION

Accordingly, it is an objective of the present invention to provide a therapeutic agent for cerebral ischemic injury, which has a lower risk of side effects and is useful for rapidly increasing the concentration of L-histidine in blood. The inventors of the present invention have focused on L-histidine and have studied a method for rapidly increasing the L-histidine concentration in blood, having a lower risk of side effects. As a result, the inventors have found that a specific histidine dipeptide that is an L-histidine derivative has high solubility in water and high stability, and that it has no side effects, thereby completing the present invention. That is to say, in order to achieve the aforementioned objective, in one aspect of the present invention, there is provided a therapeutic agent for cerebral ischemic injury, which contains at least one selected from L-alanyl-L-histidine and glycyl-L-histidine as an active ingredient.

### BRIEF DESCRIPTION OF THE DRAWINGS

Fig. 1 is a graph showing the time course of the L-histidine concentration in blood in each of three administration groups to which different L-histidine derivatives were administered.
Fig. 2 is a graph showing the influence of glycyl-L-histidine administration on cerebral ischemic injury during cerebral ischemia reperfusion.

### BEST MODE FOR CARRYING OUT THE INVENTION

One embodiment of the present invention will be described below.

The therapeutic agent for cerebral ischemic injury in the present embodiment contains, as an active ingredient, at least one selected from L-alanyl-L-histidine and glycyl-L-histidine, which are histidine derivatives. L-alanyl-L-histidine is a dipeptide (H-Ala-His-OH; molecular weight, 226.24; CAS No. 3253-17-6) in which L-alanine binds to L-histidine via a peptide bond. Glycyl-L-histidine is a dipeptide (H-Gly-His-OH; molecular weight, 212.21; CAS No. 2489-13-6) in which glycine binds to L-histidine via a peptide bond. Both L-alanyl-L-histidine and glycyl-L-histidine have a solubility in water (25°C) of 3.5 mol/L or greater, which is higher than that of L-histidine.

L-alanyl-L-histidine and glycyl-L-histidine can be obtained by a publicly known method. For example, such L-alanyl-L-histidine and glycyl-L-histidine may be synthesized by a chemical or biological technique, or commercially available products may be used. Synthesis according to a biological technique is carried out, for example, using publicly known enzyme or microorganisms. More specifically, these dipeptides may be produced by hydrolyzing a polypeptide having a known sequence with dipeptidase.

The therapeutic agent for cerebral ischemic injury preferably has a dosage form as an injection for intravenous administration. When the therapeutic agent for cerebral ischemic injury having a dosage form as an injection is intravenously administered, the blood concentration of an active ingredient contained in the therapeutic agent for cerebral ischemic injury is rapidly increased. As a result, the effects of the therapeutic agent for cerebral ischemic injury are rapidly and sufficiently exhibited. The therapeutic agent for cerebral ischemic injury having a dosage form as an injection for intravenous administration contains a solvent and at least one selected from L-alanyl-L-histidine and glycyl-L-histidine dissolved in the solvent. The solvent is preferably water or a solvent containing water. That is, the therapeutic agent for cerebral ischemic injury having a dosage form as an injection for intravenous administration is preferably an aqueous agent. Water used as a solvent is preferably sterilized water, and more preferably water for injection purpose that does not contain a pyrogenous substance. As a solvent containing water, solvents publicly known in the pharmaceutical preparation field, such as a normal saline, a dextrose solution, or a lactate Ringer's solution can be used. The therapeutic agent for cerebral ischemic injury having a dosage form as an injection for intravenous administration can be produced, for example, by dissolving at least one selected from L-alanyl-L-histidine and glycyl-L-histidine in a solvent, and then, as necessary, carrying out filtration, filling into a vessel, and a sterilization treatment.

The therapeutic agent for cerebral ischemic injury having a dosage form as an injection for intravenous administration may be dried by a publicly known method such as freeze-drying or vacuum drying, so as to convert it to a dosage form as powders or granules. The obtained therapeutic agent for cerebral ischemic injury having a dosage form as powders or granules can be dissolved in a solvent to prepare an injection, and it can be then used. That is to say, the therapeutic agent for cerebral ischemic injury may be provided in the dosage form of powders or granules, which are to be dissolved in a solvent when used. The therapeutic agent for cerebral ischemic injury having such dosage form as powders or granules may be combined with a solvent such as water for injection purpose, a normal saline, or a dextrose solution, and it may be provided as a kit product.

The therapeutic agent for cerebral ischemic injury having a dosage form as an injection for intravenous administration has a pH value of preferably pH 5 to 8.5, and more preferably pH 6 to 8.0.

The therapeutic agent for cerebral ischemic injury having a dosage form as an injection for intravenous administration is placed in an infusion container or a prefilled syringe, for example. Examples of such infusion container include a soft infusion bag made of synthetic resin and a glass bottle.

The therapeutic agent for cerebral ischemic injury having a dosage form as an injection for intravenous administration contains at least one selected from L-alanyl-L-histidine and glycyl-L-histidine at a concentration of preferably 0.3 to 3.5 mol/L, more preferably 0.6 to 2.1 mol/L, further preferably 1 to 1.7 mol/L, and particularly preferably 1 to 1.5 mol/L, although it depends on intended use.

The therapeutic agent for cerebral ischemic injury may further contain publicly known additives such as a buffer, an antioxidant, a soothing agent, and a pH adjuster, in addition to at least one selected from L-alanyl-L-histidine and glycyl-L-histidine. Examples of a buffer include a phosphate buffer, a citric acid buffer, a tartaric acid buffer, and an acetate buffer. Examples of an antioxidant include sodium sulfite, sodium hydrogen sulfite, sodium ascorbate, and sodium thiosulfate. Examples of a soothing agent include polyhydric alcohols such as glycerin and propylene glycol, lidocaine hydrochloride, and benzyl alcohol. Acid used as a pH adjuster may be either organic acid or inorganic acid. Examples of such acid include ascorbic acid, hydrochloric acid, gluconic acid, acetic acid, lactic acid, boric acid, phosphoric acid, sulfuric acid, tartaric acid, and citric acid. Examples of a base used as a pH adjuster include potassium hydroxide, calcium hydroxide, sodium hydroxide, and magnesium hydroxide.

When the therapeutic agent for cerebral ischemic injury having a dosage form as an injection for intravenous administration is produced, first, at least one selected from L-alanyl-L-histidine and glycyl-L-histidine, together with additives, as necessary, is dissolved in a solvent. Such at least one selected from L-alanyl-L-histidine and glycyl-L-histidine and additives may be simultaneously mixed into a solvent. Otherwise, such at least one selected from L-alanyl-L-histidine and glycyl-L-histidine may be added into a solvent, before or after addition of additives. The thus obtained solution is then subjected to a sterilization treatment such as filtration sterilization using a membrane filter, pressure heat sterilization using an autoclave, or heat sterilization according to an intermittent sterilization method. A preferred sterilization treatment is filtration sterilization because it has no fear of thermal denaturation. After completion of such sterilization treatment, the solution preferably has a pH value ranging from pH 5 to 8.5.

A method for administering the therapeutic agent for cerebral ischemic injury is not particularly limited. Such administration method is selected, as appropriate, depending on a dosage form, the age of a patient, the sex thereof, and the degree of disease, for example. The therapeutic agent for cerebral ischemic injury may be prepared for intravenous administration by mixing at least one selected from L-alanyl-L-histidine and glycyl-L-histidine into a transfusion solution. The type of a transfusion solution used is not particularly limited. Examples of such transfusion solution used include a dextrose injection solution, a xylitol injection solution, a D-mannitol injection solution, a fructose injection solution, a normal saline, a dextran 40 injection solution (a bloodstream improver), a dextran 70 injection solution (a plasma extender), an amino acid injection solution, a Ringer's solution, and a lactate Ringer's solution.

The dose of the therapeutic agent for cerebral ischemic injury is selected, as appropriate, depending on the administration method, a dosage form, the age of a patient, the sex thereof, and the degree of disease, for example. The dose is preferably approximately 0.1 to 3 g/kg/day in terms of L-histidine. The therapeutic agent for cerebral ischemic injury having a dosage form as an injection for intravenous administration may be administered by either an intravenous administration method applied once or several times per day, or a continuous intravenous infusion.

The therapeutic agent for cerebral ischemic injury is preferably used for the treatment of cerebral ischemic injury. More specifically, the therapeutic agent for cerebral ischemic injury is preferably administered when brain tissue is damaged by cerebral ischemia, namely when brain cells are necrosed or are in a condition close to such necrosis. Otherwise, the therapeutic agent for cerebral ischemic injury is preferably administered when brain cells are recovering from a cerebral ischemic condition, so that the agent prevents free radicals or inflammatory reactions that occur during reperfusion caused by the recovery of the cells from such cerebral ischemic condition from destroying peripheral brain tissue. Otherwise, the therapeutic agent for cerebral ischemic injury may also be administered during the recovery period from cerebral ischemic injury, in order to prevent further progression of the necrosis of brain cells.

According to the present embodiment, the following advantages can be obtained.
(1) The therapeutic agent for cerebral ischemic injury in the present embodiment contains at least one selected from L-alanyl-L-histidine and glycyl-L-histidine as an active ingredient. Since these L-histidine derivatives have solubility in a solvent such as water, which is higher than that of L-histidine, they are easily administered at high concentrations into living bodies.
(2) The therapeutic agent for cerebral ischemic injury acts to effectively improve cerebral ischemic injury, without having a risk of side effects.
(3) The therapeutic agent for cerebral ischemic injury having a dosage form as an injection for intravenous administration is effective for rapidly increasing the concentration of L-histidine in blood, when administered via intravenous administration. In addition, the therapeutic agent for cerebral ischemic injury having a dosage form as an injection for intravenous administration is also effective for continuously administering L-histidine.
(4) When the therapeutic agent for cerebral ischemic injury having a dosage form as an injection for intravenous administration contains at least one selected from L-alanyl-L-histidine and glycyl-L-histidine at a concentration of 0.3 to 3.5 mol/L, preferably 0.6 to 2.1 mol/L, and more preferably 1 to 1.5 mol/L, it is able to reduce a risk of side effects, and it is also able to rapidly increasing the L-histidine concentration in blood to an effective level by administering the therapeutic agent for cerebral ischemic injury in a relatively small amount.
(5) L-alanyl-L-histidine and glycyl-L-histidine have relatively high solubility in a solvent. As a result, the amount of a solvent used in the therapeutic agent for cerebral ischemic injury having a dosage form as an injection for intravenous administration can be reduced. This is advantageous for preventing the disruption of water-electrolyte balance in a living body or a transient increase in blood pressure, which is caused by the solvent contained in the therapeutic agent for cerebral ischemic injury administered.

The aforementioned embodiment may be modified as follows.

Instead of having a dosage form as an injection for intravenous administration, the therapeutic agent for cerebral ischemic injury may have a dosage form such as an intraarterial injection, a hypodermic injection, an intradermal injection, an intramuscular injection, an intraspinal injection, an intraperitoneal injection, or an orally-administered agent. In this case as well, it is expected that the therapeutic agent for cerebral ischemic injury is useful for increasing the L-histidine concentration in blood.

Targets to which the therapeutic agent for cerebral ischemic injury is to be administered are not limited to humans. This agent may also be administered to breeding animals including livestock animals (e.g. a horse, a bovine, a swine) and domestic poultries (e.g. a chicken), and also to pet animals such as a dog, a cat, a rat, and a mouse.

Next, the present invention will be more specifically described in the following production examples of producing the therapeutic agent for cerebral ischemic injury of the present invention, and test examples using the therapeutic agents for cerebral ischemic injury obtained in the production examples.

### Production Example 1

29.2 g of L-alanyl-L-histidine (manufactured by Bachem) was dissolved in water for injection purpose to prepare 100 mL (total amount) of solution. This solution was subjected to sterilization filtration, and it was then poured into glass vials (10 mL each per vial). Thereafter, each glass vial was hermetically sealed, so as to produce a therapeutic agent for cerebral ischemic injury having a dosage form as an injection. The concentration of L-alanyl-L-histidine in this therapeutic agent for cerebral ischemic injury was 1.29 mol/L (L-histidine concentration: 20% (W/V)).

### Production Example 2

A therapeutic agent for cerebral ischemic injury having a dosage form as an injection was produced in the same manner as that of Production Example 1, except that 27.4 g of glycyl-L-histidine (manufactured by Bachem) was used, instead of 29.2 g of L-alanyl-L-histidine. The concentration of glycyl-L-histidine in this therapeutic agent for cerebral ischemic injury was 1.29 mol/L (L-histidine concentration: 20% (W/V)).

### Production Example 3

A therapeutic agent for cerebral ischemic injury having a dosage form as an injection was produced in the same manner as that of Production Example 1, except that 14.6 g of L-alanyl-L-histidine (manufactured by Bachem) and 13.7 g of glycyl-L-histidine (manufactured by Bachem) were used, instead of 29.2 g of L-alanyl-L-histidine. The total concentration of L-alanyl-L-histidine and glycyl-L-histidine in this therapeutic agent for cerebral ischemic injury was 1.29 mol/L (L-histidine concentration: 20% (W/V)).

### Production Example 4

16.41 g of glycyl-L-histidine (manufactured by Bachem) was dissolved in water for injection purpose to prepare 120 mL (total amount) of solution. This solution was subjected to sterilization filtration, and it was then poured into glass vials (10 mL each per vial). Thereafter, each glass vial was hermetically sealed, so as to produce a therapeutic agent for cerebral ischemic injury having a dosage form as an injection. The concentration of glycyl-L-histidine in this therapeutic agent for cerebral ischemic injury was 0.64 mol/L (L-histidine concentration: 10% (W/V)).

### Production Example 5

3.39 g of L-alanyl-L-histidine (manufactured by Bachem) was dissolved in water for injection purpose to prepare 10 mL (total amount) of solution. This solution was subjected to sterilization filtration, and it was then poured into glass syringes (1 mL each per syringe). Thereafter, each glass syringe was hermetically sealed, so as to produce a therapeutic agent for cerebral ischemic injury having a dosage form as an injection. The concentration of L-alanyl-L-histidine in this therapeutic agent for cerebral ischemic injury was 1.5 mol/L (L-histidine concentration: 23.3% (W/V)).

### Production Example 6

3.61 g of glycyl-L-histidine (manufactured by Bachem) was dissolved in water for injection purpose to prepare 10 mL (total amount) of solution. This solution was subjected to sterilization filtration, and it was then poured into glass syringes (1 mL each per syringe). Thereafter, each glass syringe was hermetically sealed, so as to produce a therapeutic agent for cerebral ischemic injury having a dosage form as an injection. The concentration of glycyl-L-histidine in this therapeutic agent for cerebral ischemic injury was 1.7 mol/L (L-histidine concentration: 26.4% (W/V)).

### Production Example 7

4.75 g of L-alanyl-L-histidine (manufactured by Bachem) was dissolved in water for injection purpose to prepare 10 mL (total amount) of solution. This solution was subjected to sterilization filtration, and it was then poured into glass syringes (1 mL each per syringe). Thereafter, each glass syringe was hermetically sealed, so as to produce a therapeutic agent for cerebral ischemic injury having a dosage form as an injection. The concentration ofL-alanyl-L-histidine in this therapeutic agent for cerebral ischemic injury was 2.1 mol/L (L-histidine concentration: 32.6% (W/V)).

### Production Example 8

3.18 g of glycyl-L-histidine (manufactured by Bachem) was enclosed in one chamber of a two-chamber plastic bag, and 50 mL of distilled water for injection purpose was enclosed in the other chamber thereof, so as to produce a therapeutic agent for cerebral ischemic injury having a dosage form as an injection, which was to be dissolved when used. The concentration of glycyl-L-histidine in a solution, which was obtained by connecting the two chambers and mixing the contents in the two chambers, was 0.3 mol/L (L-histidine concentration: 4.7% (W/V)).

### Test Example 1: Confirmation of effect of suppressing cerebral ischemic injury during cerebral ischemia-reperfusion

### <Production of model of middle cerebral artery occlusion and subsequent

### reperfusion>

Approximately 9-week-old Wistar male rats were used. The method of Koizumi et al. (Nou-socchu (Cerebral Apoplexy), Vol. 8, pp. 1-8, 1986) was applied to the rats under halothane anesthesia, so as to produce models of middle cerebral artery occlusion and subsequent reperfusion. Specifically, each rat was fixed under natural aspiration conditions under anesthesia with gas (initial anesthesia: performed in room air containing 3% halothane; continuous anesthesia: performed in room air containing 1% halothane), and the neck of the rat was then subjected to median incision. Subsequently, the left common carotid and external carotid artery was exfoliated from surrounding connective tissues, and it was then ligated with a ligature. After that, the internal carotid artery was exfoliated from surrounding connective tissues, and it was then ligated with a ligature. Further, using scissors, a hole was made on a site close to the branch point between the left external carotid artery and the internal carotid artery, and a silicon-coated portion of a nylon plug was then inserted into the internal carotid artery at a depth of 15 to 16 mm. Thereby, the tip of the inserted nylon plug exceeded the middle cerebral artery portion and reached the anterior cerebral artery, so that the inlet of the middle cerebral artery was occluded with the silicon-coated portion of the plug. Thereafter, a small extent of the end of a nylon thread was left out of the blood vessel, the area was then ligated, and the neck was then sutured.

Approximately 15 minutes after the surgery, and before the reperfusion of the middle cerebral artery, the presence or absence of nervous symptoms was checked. Animals having nervous symptoms were selected. From among the selected animals, animals having a high rectal temperature 90 minutes after the surgery were further selected. The thus selected animals were used as test animals.

### <Administration of agent>

Ninety minutes after occlusion of the middle cerebral artery, the aforementioned artery was reperfused. Immediately after such reperfusion and 6 hours after the reperfusion, the therapeutic agent for cerebral ischemic injury having a dosage form as an injection, which was obtained in the above described Production Example 1, was administered to the test animals (experiment group). Reperfusion was carried out by removing the nylon plug that occluded the middle cerebral artery. The therapeutic agent for cerebral ischemic injury was administered into the caudal vein of each test animal, using a syringe pump prepared by fixing a 50-mL polypropylene disposable syringe with a winged intravenous injection needle, at a rate of 60 mL/kg/hr for 10 minutes. The test animals were free to consume a diet (number of rats (n) = 10). A control group, to which a normal saline was administered in the same above manner, instead of the therapeutic agent for cerebral ischemic injury of Production Example 1, was prepared. Further, a comparison group, to which Edaravone (product name Radicut, manufactured by Mitsubishi Tanabe Pharma Corporation) was administered via bolus injection at a dose of 3 mg/kg, immediately after the reperfusion and 30 minutes after the reperfusion, was also prepared.

### <Measurement of volume of cerebral infarct>

On the day after the administration of the agent, a test animal was anesthetized with ether, and the brain was then excised. The left side of the brain was divided into 6 sections with a thickness of approximately 2 mm. A slice of brain section was placed, with a surface to be measured down, into each well of a 12-well plate, which had been filled with a 2% TTC solution prepared by dissolving 2,3,5-triphenyltetrazolium chloride (TTC) in a phosphate buffered saline. It was then stained in a dark place at a room temperature. After staining, the measurement surface of each brain section was photographed with a digital camera, and the thus photographed image was then incorporated into image analysis software. Thus, the area of an infarct on the measurement surface of each brain section (the site in which brain tissue was considered to be necrosed) was measured, and the obtained value was then multiplied by the thickness of the brain section, so as to obtain the volume of an infarct in each brain section. As an infarction rate, the ratio (percentage) of the total volume of infarcts to the entire volume of the left side of the brain was calculated, and a mean value in each group was then obtained. The results are shown in Table 1.

**Table 1**

| | Infarction rate (mean value) |
|---|---|
| Control group | 49% |
| Comparison group | 38% |
| Experiment group | 20% |

From the above results, it was found that, when compared with the control group and the comparison group, cerebral ischemic injury was remarkably suppressed during the cerebral ischemia-reperfusion in the experiment group. That is, it was demonstrated that the therapeutic agent for cerebral ischemic injury obtained in Production Example 1 acts to suppress the onset and progression of cerebral infarction.

### Test Example 2: Measurement of L-histidine concentration in blood and identification of side effects

### 7- to 9-week-old Wistar male rats were divided into the following 8 administration groups. The number of rats in each group was set at 4.

### (First administration group)

The therapeutic agent for cerebral ischemic injury obtained in Production Example 1 was administered into the caudal vein of each rat at a rate of 60 mL/kg/hr for 10 minutes.

### (Second administration group)

The therapeutic agent for cerebral ischemic injury obtained in Production Example 1 was diluted by a factor of 2, and the thus diluted solution was administered into the caudal vein of each rat at a rate of 60 mL/kg/hr for 10 minutes.

### (Third administration group)

The therapeutic agent for cerebral ischemic injury obtained in Production Example 2 was diluted by a factor of 2, and the thus diluted solution was administered into the caudal vein of each rat at a rate of 60 mL/kg/hr for 10 minutes.

### (Fourth administration group)

L-histidine hydrochloride was dissolved in water for injection purpose, and the obtained solution was then adjusted to pH 6.0 with a sodium hydroxide aqueous solution. The obtained injection solution (L-histidine concentration: 10% (W/V)) was administered into the caudal vein of each rat at a rate of 60 mL/kg/hr for 10 minutes.

### (Fifth administration group)

L-histidine was added to water for injection purpose, and acetic acid was further added thereto, so as to dissolve L-histidine in the solution. The obtained solution was adjusted to pH 6.0 with a sodium hydroxide aqueous solution. The obtained injection solution (L-histidine concentration: 10% (W/V)) was administered into the caudal vein of each rat at a rate of 60 mL/kg/hr for 10 minutes.

### (Sixth administration group)

L-histidine was added to water for injection purpose, and citric acid was further added thereto, so as to dissolve L-histidine in the solution. The obtained solution was adjusted to pH 6.0 with a sodium hydroxide aqueous solution. The obtained injection solution (L-histidine concentration: 10% (W/V)) was administered into the caudal vein of each rat at a rate of 60 mL/kg/hr for 10 minutes.

### (Seventh administration group)

β-alanyl-L-histidine was added to water for injection purpose, and the obtained solution was then adjusted to pH 7 with hydrochloric acid. The obtained injection solution (L-histidine concentration: 10% (W/V)) was administered into the caudal vein of each rat at a rate of 60 mL/kg/hr for 10 minutes.

### (Eighth administration group)

N-acetyl-L-histidine was added to water for injection purpose, and the obtained solution was then adjusted to pH 7 with a sodium hydroxide aqueous solution. The obtained injection solution (L-histidine concentration: 8% (W/V)) was administered into the caudal vein of each rat at a rate of 60 mL/kg/hr for 10 minutes.

After administration of the agent, blood was collected from each rat at regular time intervals, and the concentration of L-histidine (monomer) in blood was then measured using a high speed amino acid analyzer L-8800A (manufactured by Hitachi High-Technologies Corporation). As a result, it was found that the concentration of L-histidine in blood reached maximum, 15 minutes after the administration of the agent in all cases. In addition, the body temperature, blood pressure, and cardiac rate of each test animal were measured during the period from before administration of the agent until after the administration of the agent, and at the same time, the appearance of each test animal was observed, so as to examine the presence or absence of side effects caused by administration of the agent. The L-histidine concentration in blood was measured 15 minutes after the administration of the agent, and the presence or absence of side effects caused by such administration of the agent was examined. The results are shown in Table 2.

**Table 2**

| | | |
|---|---|---|
| | L-histidine concentration in blood (mean value) | Side effects |
| 1^{st} administration group | 19.3 mM | None |
| 2^{nd} administration group | 5.5 mM | None |
| 3^{rd} administration group | 6.2 mM | None |
| 4^{th} administration group | 14.5 mM | Decrease in blood pressure Cessation of breathing during administration of agent in 2 cases |
| 5^{th} administration group | Not be measurable due to death of test animal | Decrease in blood pressure Cessation of breathing immediately after administration of agent in 4 cases |
| 6^{th} administration group | Not be measurable due to death of test animal | Decrease in blood pressure Cessation of breathing during administration of agent in 4 cases |
| 7^{th} administration group | 0.30 mM | Cessation of breathing immediately after administration of agent in 2 cases |
| 8^{th} administration group | 0.25 mM | None |

As shown in Table 2, it was found that the concentration of L-histidine in blood was effectively increased in the first to third administration groups, without having side effects. Accordingly, it is expected that the therapeutic agents for cerebral ischemic injury obtained in Production Examples 1 and 2 or the diluted products thereof can be preferably used for the treatment of cerebral infarction. By comparing the results of the first administration group with the results of the second administration group, it was found that the L-histidine concentration in blood was increased by a factor of 3 to 3.5, when the concentration of L-alanyl-L-histidine administered was increased by a factor of 2. On the other hand, with regard to the fourth to seventh administration groups, it was found that side effects were caused by administration of the agents. Moreover, with regard to the seventh and eighth administration groups, it was found that the L-histidine concentration in blood was not effectively increased.

### Test Example 3: Determination of solubility of L-histidine derivatives (dipeptides) in solvent

Injection solutions having pH 6 to 7 were attempted to be produced using the following 9 types of dipeptides containing L-histidine as a part of constitutional amino acids: (1) L-histidyl-L-glutamic acid; (2) L-histidyl-L-tyrosine; (3) L-histidyl-L-proline; (4) L-histidyl-L-phenylalanine; (5) L-histidyl-L-leucine; (6) L-histidyl glycine; (7) L-histidyl-L-methionine; (8) L-histidyl-L-serine; and (9) L-glutamyl-L-histidine. However, all of these dipeptides could not be dissolved at a concentration of 0.3 mol/L (L-histidine concentration: 4.67% (W/V)) or more. From these results, it was expected that the 9 types of dipeptides would not be suitable for the purpose of rapidly increasing the L-histidine concentration in blood via injection administration.

### Test Example 4: Comparison among L-histidine concentrations in blood by administration of different types of L-histidine derivatives (dipeptides)

### 7- to 8-week-old Wister male rats were divided into the following 3 administration groups. The number of rats in each group was set at 2 or 3.

### (L-alanyl-L-histidine administration group)

1.00 g of L-alanyl-L-histidine (manufactured by Bachem) was dissolved in water for injection purpose to prepare 13.70 mL (total amount) of solution. This solution was subjected to sterilization filtration, and it was then poured into syringes (10 mL each per syringe), so as to produce a therapeutic agent for cerebral ischemic injury having a dosage form as an injection. The concentration of L-alanyl-L-histidine in this therapeutic agent for cerebral ischemic injury was 0.32 mol/L (L-histidine concentration: 5% (W/V)). The obtained therapeutic agent for cerebral ischemic injury was administered into the caudal vein of each rat at a rate of 60 mL/kg/hr for 10 minutes.

### (L-histidyl-L-glycine administration group)

1.00 g of L-histidyl-L-glycine (manufactured by Bachem) was dissolved in water for injection purpose to prepare 14.60 mL (total amount) of solution. This solution was subjected to sterilization filtration, and it was then poured into syringes (10 mL each per syringe), so as to produce a therapeutic agent for cerebral ischemic injury having a dosage form as an injection. The concentration of L-histidyl-L-glycine in this therapeutic agent for cerebral ischemic injury was 0.32 mol/L (L-histidine concentration: 5% (W/V)). The obtained therapeutic agent for cerebral ischemic injury was administered into the caudal vein of each rat at a rate of 60 mL/kg/hr for 10 minutes.

### (L-histidyl-L-glutamic acid administration group)

1.00 g of L-histidyl-L-glutamic acid (manufactured by Bachem) was dissolved in water for injection purpose to prepare 10.90 mL (total amount) of solution. This solution was subjected to sterilization filtration, and it was then poured into syringes (10 mL each per syringe), so as to produce a therapeutic agent for cerebral ischemic injury having a dosage form as an injection. The concentration of L-histidyl-L-glutamic acid in this therapeutic agent for cerebral ischemic injury was 0.32 mol/L (L-histidine concentration: 5% (W/V)). The obtained therapeutic agent for cerebral ischemic injury was administered into the caudal vein of each rat at a rate of 60 mL/kg/hr for 10 minutes.

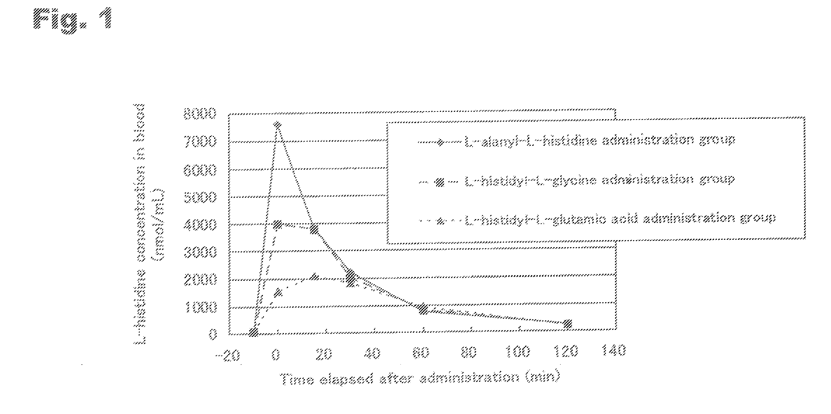

Before and after the administration of the agent, blood was collected from each rat at regular time intervals, and the concentration of L-histidine (monomer) in blood was then measured using a high speed amino acid analyzer L-8800A (manufactured by Hitachi High-Technologies Corporation). The results are shown in Table 3 and Fig. 1.

**Table 3**

| | L-histidine concentration in blood (nmol/mL) | | |
|---|---|---|---|
| Time elapsed after administration of agent (min) | L-alanyl-L-histidine administration group | L-histidyl-L-glycine administration group | L-histidyl-L-glutamic acid administration group |
| -10 | 76.67 | 74.73 | 78.40 |
| 0 | 7588.27 | 3995.57 | 1546.15 |
| 15 | 3802.80 | 3798.87 | 2090.10 |
| 30 | 2198.93 | 2019.47 | 1827.15 |
| 60 | 770.17 | 833.93 | 926.80 |
| 120 | 268.77 | 244.03 | 233.20 |

As shown in Table 3 and Fig. 1, the concentration of L-histidine in blood reached maximum during the period from immediately after (0 min) to 15 minutes after completion of the administration of the agent in all administration groups, and thereafter, the L-histidine concentration in blood was rapidly decreased. It was found that, among the three administration groups, the L-histidine concentration in blood was increased to the highest level in the first administration group. Moreover, it was also found that the L-histidine concentration in blood immediately after completion of the administration of the agent in the first administration group was approximately 2 times higher than that of the second administration group, and was approximately 5 times higher than that of the third administration group. Side effects (dead cases) caused by the administration of the agent were not observed in any administration groups. From these results, it was found that L-alanyl-L-histidine was most effective for rapidly increasing the L-histidine concentration in blood without having side effects.

Test Example 5: Confirmation of effect of suppressing cerebral ischemic injury during cerebral ischemia-reperfusion

### <Production of model of middle cerebral artery occlusion and subsequent reperfusion>

Approximately 7-week-old Wistar male rats were used. The method of Koizumi et al. (Nou-socchu (Cerebral Apoplexy), Vol. 8, pp. 1-8, 1986) was applied to the rats under halothane anesthesia, so as to produce models of middle cerebral artery occlusion and subsequent reperfusion. Specifically, each rat was fixed under natural aspiration conditions under anesthesia with gas (initial anesthesia: performed in room air containing 3% halothane; continuous anesthesia: performed in room air containing 1% halothane), and the neck of the rat was then subjected to median incision. Subsequently, the right common carotid and external carotid artery was exfoliated from surrounding connective tissues, and it was then ligated with a ligature. After that, the internal carotid artery was exfoliated from surrounding connective tissues, and it was then ligated with a ligature. Further, using scissors, a hole was made on a site close to the branch point between the right external carotid artery and the internal carotid artery, and a silicon-coated portion of a nylon plug was then inserted into the internal carotid artery at a depth of 17 to 18 mm. Thereby, the tip of the inserted nylon plug exceeded the middle cerebral artery portion and reached the anterior cerebral artery, so that the inlet of the middle cerebral artery was occluded with the silicon-coated portion of the plug. Thereafter, a small extent of the end of a nylon thread was left out of the blood vessel, the area was then ligated, and the neck was then sutured.

Approximately 15 minutes after the surgery, and before the reperfusion of the middle cerebral artery, the presence or absence of nervous symptoms was checked. Animals having nervous symptoms were selected. From among the selected animals, animals having a high rectal temperature 90 minutes after the surgery were further selected. The thus selected animals were used as test animals.

### <Administration of agent>

120 minutes after occlusion of the middle cerebral artery, the aforementioned artery was reperfused. Immediately after such reperfusion and 6 hours after the reperfusion, the therapeutic agent for cerebral ischemic injury having a dosage form as an injection, which was obtained in the above described Production Example 4, was administered to the test animals (experiment group). Reperfusion was carried out by removing the nylon plug that occluded the middle cerebral artery. The therapeutic agent for cerebral ischemic injury was administered into the caudal vein of each test animal, using a syringe pump prepared by fixing a 50-mL polypropylene disposable syringe with a winged intravenous injection needle, at a rate of 60 mL/kg/hr for 10 minutes. The test animals were free to consume a diet (number of rats (n) = 8 to 10). A control group, to which a normal saline was administered in the same above manner, instead of the therapeutic agent for cerebral ischemic injury, was also prepared.

### <Measurement of volume of cerebral infarct>

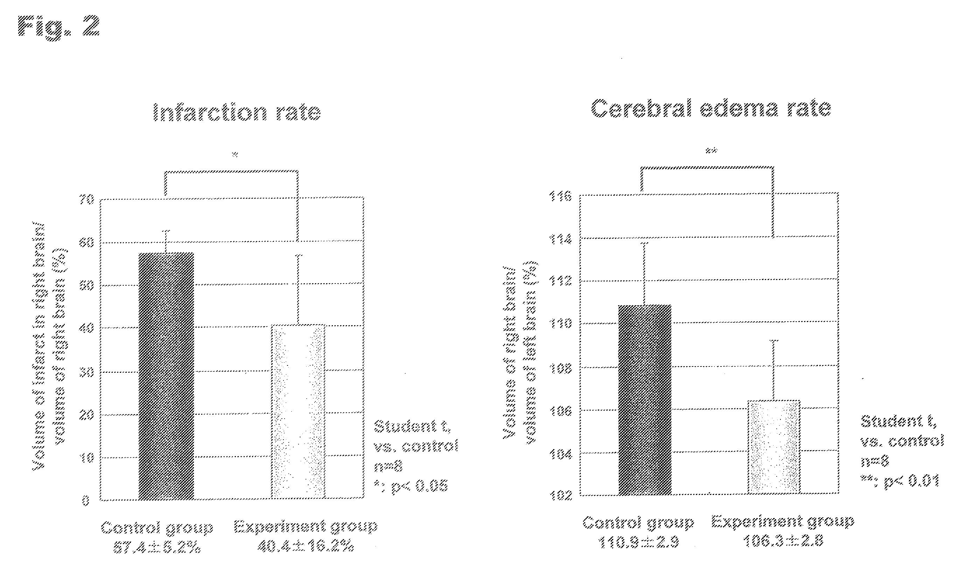

On the day after the administration of the agent (24 hours after cerebral ischemia-reperfusion), a test animal was anesthetized with ether, and the brain was then excised. The right side of the brain was divided into 6 sections with a thickness of approximately 2 mm. A slice of brain section was placed, with a surface to be measured down, into each well of a 12-well plate, which had been filled with a 2% TTC solution prepared by dissolving 2,3,5-triphenyltetrazolium chloride (TTC) in a phosphate buffered saline. It was then stained in a dark place at a room temperature. After staining, the measurement surface of each brain section was photographed with a digital camera, and the thus photographed image was then incorporated into image analysis software. Thus, the area of an infarct on the measurement surface of each brain section (the site in which brain tissue was considered to be necrosed) was measured, and the obtained value was then multiplied by the thickness of the brain section, so as to obtain the volume of an infarct in each brain section. As an infarction rate, the ratio (percentage) of the total volume of infarcts to the entire volume of the right side of the brain was calculated, and a mean value in each group was then obtained. Moreover, as a cerebral edema rate, the ratio (percentage) of the entire volume of the right side of the brain to the entire volume of the left side of the brain was calculated, and a mean value in each group was then obtained. The results are shown in Fig. 2.

From the results shown in Fig. 2, it was found that, when compared with the control group, cerebral ischemic injury (infarction rate and cerebral edema rate) was remarkably suppressed during the cerebral ischemia-reperfusion in the experiment group. That is, it was demonstrated that the therapeutic agent for cerebral ischemic injury obtained in Production Example 4 acts to suppress the onset and progression of cerebral infarction.

## Claims

1. A therapeutic agent for cerebral ischemic injury, **characterized by** containing at least one selected from L-alanyl-L-histidine and glycyl-L-histidine as an active ingredient.

2. The therapeutic agent for cerebral ischemic injury according to claim 1, having a dosage form as an injection for intravenous administration.

3. The therapeutic agent for cerebral ischemic injury according to claim 2, being aqueous and containing at least one selected from L-alanyl-L-histidine and glycyl-L-histidine at a concentration of 0.3 to 3.5 mol/L.

4. The therapeutic agent for cerebral ischemic injury according to claim 3, containing at least one selected from L-alanyl-L-histidine and glycyl-L-histidine at a concentration of 0.6 to 2.1 mol/L.
